# EUROPEAN PATENT SPECIFICATION

(11) **EP 1 790 733 B1**
(45) Date of publication and mention of the grant of the patent: **04.08.2010**
(21) Application number: 05780915.4
(22) Date of filing: 24.08.2005
(51) Int. Cl.: C12Q 1/02, C12Q 1/34, C12M 1/34

(54) **SCREENING METHOD AND SCREENING APPARATUS USING MICRO-CHAMBER ARRAY**
SCREENING-VERFAHREN UND SCREENING-VORRICHTUNG UNTER VERWENDUNG EINES MIKROKAMMER-ARRAYS
PROCÉDÉ DE CRIBLAGE ET APPAREIL DE CRIBLAGE À L'AIDE D'UN RÉSEAU DE MICROCHAMBRES

(30) Priority: 24.08.2004 JP 2004244275
(43) Date of publication of application: 30.05.2007
(73) Proprietor: Suntory Holdings Limited, Kita-ku, Osaka-shi Osaka 530-8203 (JP)
(72) Inventor: UEDA, Mitsuyoshi, 6650033 (JP); NAKAO, Masahiro, 6170827 (JP); ASAMI, Maki, Kyoto-shi, Kyoto 6160016 (JP); OCHIAI, Misa, Mishima-gun, Osaka 6180001 (JP); FUKAMI, Harukazu, Kyoto-shi, Kyoto 6018373 (JP)
(74) Representative: Vossius & Partner
(86) International application number: PCT/JP2005/015342
(87) International publication number: WO 2006/022293

(56) References cited:
- WO-A1-86/05206
- JP-A- 2 046 280
- US-A1- 2002 132 364
- ANGENENDT P ET AL: "CELL-FREE PROTEIN EXPRESSION AND FUNCTIONAL ASSAY IN NANOWELL CHIP FORMAT" ANALYTICAL CHEMISTRY, AMERICAN CHEMICAL SOCIETY. COLUMBUS, US, vol. 76, no. 7, 1 April 2004 (2004-04-01), pages 1844-1849, XP001196723 ISSN: 0003-2700
- SCHULLEK J R ET AL: "A high density screening format for encoded combinatorial libraries: assay miniturization and its application enzymatic reactions" ANALYTICAL BIOCHEMISTRY, ACADEMIC PRESS, NEW YORK, NY, US, vol. 246, no. 1, 1 March 1997 (1997-03-01), pages 20-29, XP002997363 ISSN: 0003-2697

## Description

### TECHNICAL FIELD

The invention relates to a method for screening a protein having an enzymatic activity or a microorganism producing a protein having an enzymatic activity by using a micro-camber array. Further, the invention relates to a method for verifying whether a microorganism expresses a protein having an enzymatic activity in the surface layer of a cell membrane or verifying secretion of the protein having an enzymatic activity.

### BACKGROUND ART

In the conventional screening technology of microorganisms having useful functions (for example, microorganisms producing useful proteins), a standard method comprises scattering the microorganisms on an agar-agar plate and checking whether they are multiplied by applying a certain selected condition, or adding a substrate and checking whether, for example, a halo is formed. With such a method, the target microorganism is screened. However, the problems associated with such a method are that a large number of agar-agar plates are required and producing agar-agar plates and scattering the microorganisms are troublesome, time-consuming, and costly operations.

Further, micro-samples will have to be used and high-speed analysis will have to be performed in the future for analyzing the functions of a larger number of genes or proteins in genomics or proteomics. DNA chips and proteochips for high-speed analysis of micro-samples have been developed to meet such a need. Furthermore, the progress in micro-machining technology has made it possible to fabricate ultra small chambers (wells). In addition, quantitative and semi-qualitative analysis was made possible by using CCD cameras and computers.

Non-patent document 1 reports a technology by which a PCR was carried out in one cell, or cell-free protein synthesis was performed, or cells were introduced and fluorescing chromogenic cells were detected by actually using such a micro chamber array technology. Further, Non-patent document 1 reports that comprehensively transformed genes were introduced in E. coli or yeast and the functions of the expressed protein could be analyzed.

As such a technology has been advanced, a high-throughput screening that enables simultaneous and fast functional analysis of a large number of genes or proteins has attracted attention. The presently employed high-throughput screening is implemented by a method by which a large number of compounds are screened by using 96 wells or 384 wells and employing an automated device. Further, Non-patent document 2 describes a technology for high-throughput screening of proteins synthesized by using a cell-free protein synthesis system.
Non-patent document 3 discloses assays in nanowells wherein the assay either involves cell-free expression of proteins down to volumes of 100 nL and detection by fluorescence or enzymatic inhibition assays and detection by fluorescence.
US 2002/132364 discloses a screening method using a 96 well microplate with a test volume of 100 nL or a 384 well microplate with a well volume of 25 µL by measuring fluorescence emitted by the cleared substrate of a hydrolytic enzyme of the cell.

Non-patent document 1: "Special issue: Combinatorial Bioengineering", Bioindustry, 2001, Vol.18, No.6, 7 to 17, 56 to 72
Non-patent document 2: Nippon Nogeikagaku Kaishi, Vol. 78, No.5, 27 to 30 (published on May 1, 2004).
Non-patent document 3: Angenendt et al., Anal. Chem. Vol. 76, No. 7, 1844-1849, 2004.

### DISCLOSURE OF INVENTION

### PROBLEMS TO BE SOLVED BY THE INVENTION

However, the volume of each chamber present in the plate with 96 wells or 384 wells is about 300 µL in the case of 96 wells and about 100 µL in the case of 384 wells. When screening is conducted by using such plates, a solution filling at least one third of the volume of each chamber is required and a matching quantity of a reagent is required. If the number of screening cycles is large, the cost increases accordingly.

Further, in recent years, it has become necessary to perform fast and inexpensive micro-screening of proteins or microorganisms having an enzymatic activity such as lipase activity from even larger libraries. If screening is performed with the above-described agar-agar plate, then the work volume and cost increase; and satisfying such a need is difficult. Further, high-throughput screening is actually conducted by using a 96 well or 384 well plate and performing an enzyme reaction, but processing larger quantities creates many problems, high cost including.

On the other hand, with a micro-chamber with an inner diameter of about 100 µm and a depth of about 10 µm, the volume thereof becomes about 80 pL and constitutes 1/100,000 that of the chamber provided in the aforementioned 96-well or 384-well plate. Further, with the micro-chamber having the aforementioned volume, about 5000 to 100,000 micro-chambers can be arranged on a plate with a surface area of 1 cm². Therefore, it can be expected that if a screening of microorganisms or proteins is performed by using a plate in which micro- chambers are arranged in the form of an array, then high-speed low-cost functional analysis can be realized.

However, although a variety of reactions can be induced inside a micro-chamber, enzyme reactions and high-throughput screening have not yet been conduced therein.

In other words, the conventional 96-well or 384-well plates were aimed at screening the enzyme inhibitors for medial applications. Because enzyme inhibitors are low-molecular compounds, the chemical library, though being large, is limited to about 1,000,000 units and the demand for micro-chamber arrays therefore has not been that strong. However, in recent years, the necessity of screening from samples containing larger volume libraries and a need for performing the screening using micro-chamber arrays were created.

Accordingly, the invention provides a method for screening a microorganism and a protein having a useful function by employing the above-described micro-chamber array. Further, the present invention provides a method capable of verifying whether the protein having an enzymatic activity is expressed in the surface layer of a cell membrane or by secretion in a cell of a microorganism such as yeast by gene recombination.

### MEANS FOR SOLVING THE PROBLEM

In order to resolve the above-described problems, the screening method in accordance with the invention comprises the steps of adding a fluorescent substrate that produces fluorescence by hydrolysis onto a base plate having a plurality of micro-chambers arranged in the form of an array, dripping a sample comprising a protein or a microorganism onto the base plate, and detecting a protein having an enzymatic activity or a microorganism producing such a protein that is present inside the micro-chamber by detecting fluorescence emitted by an enzyme reaction of the protein or the microorganism and the fluorescent substrate.

With the method for screening a protein or a microorganism in accordance with the invention, a protein having an enzymatic activity or a microorganism producing such a protein is screened by using a base plate having a plurality of micro-chambers arranged in the form of an array.

With such a screening method, using a base plate having very small micro-chambers with a volume on the order of picoliters makes it possible to perform fast and inexpensive screening from samples containing a large-volume library. Furthermore, because each micro-chamber is extremely small, a micro-amount of solution comprising the target protein or the microorganism can be extracted from the sample. As a result, in the case of microorganisms, one or several microorganisms can be taken out and they can also be caused to multiply. Therefore, microorganisms can be used in their existing state or in a multiplied state, for example, in the functional analysis performed at a stage following the screening. The library as referred to herein means all the proteins or all the microorganisms (including proteins and microorganisms other than the target proteins and microorganisms) that are contained in the sample.

Further, "the sample comprising a protein or a microorganism" also includes samples that can contain proteins or microorganisms having a certain specific enzymatic activity. In other words, the sample means a screening object of the target protein or the microorganism. Specific examples of such samples include samples taken from natural environment such as sea soil and samples containing mutant proteins or variants of microorganisms that are produced by a genetic transformation method based on the conventional well-known genetic engineering technique.

When screening is conducted herein by using a sample taken from the natural environment such as sea soil, useful microorganisms having a specific enzymatic activity can be screened from the natural world. Furthermore, using a sample containing mutant proteins or variants of microorganisms makes it possible to isolate easily the mutant proteins or variants produced from vast libraries.

In the screening method in accordance with the invention, the protein is a protein expressed in the surface layer of a cell membrane of a microorganism, and in the above-described step of detecting, the microorganism that expresses a protein having an enzymatic activity in the surface layer of a cell membrane is preferably detected by detecting a fluorescing micro-chamber from among a plurality of micro-chambers.

In other words, with the above-described screening method, the protein that is the object of screening is a protein expressed in the surface layer of a cell membrane of a microorganism such as yeast. Further, in this case, a microorganism in which a protein having an enzymatic activity being expressed in the surface layer of a cell membrane is also included in the objects of screening.

If a sample containing a microorganism in which a protein is expressed in the surface layer of a cell membrane is dripped onto a base plate, the microorganism is distributed between a plurality of micro-chambers formed on the base plate. For this reason, with the above-described screening method, the micro-chamber in which the target protein is contained can be verified by detecting the fluorescing micro-chamber.

If an enzyme reaction is implemented by introducing a substrate into a cell in the screening method in accordance with the present invention, then not only the protein expressed by secretion outside the cell or the protein expressed in the surface layer of a cell membrane, but also the protein expressed inside the cell can be screened.

The inner diameter of the micro-chamber in the screening method in accordance with the present invention is 5 to 500 µm.

If the inner diameter of the micro-chamber is within the aforementioned range of 5 to 500 µm, an extremely small volume on the order of picoliters can be obtained for the micro-chamber.

The screening method in accordance with the invention preferably further comprises a step of taking out the contents of micro-chambers where the protein is contained after the aforementioned step of detecting.

With the aforementioned take-out step, a micro amount of solution containing the target protein or a microorganism can be obtained. Despite an extremely small quantity thereof, the solution reliably contains the target protein or the microorganism. Therefore, a solution can be used upon direct multiplication, for e.g., in the functional analysis performed after the screening.

In order to resolve the above-described problems, the present invention also discloses a screening apparatus for screening a protein having an enzymatic activity and a microorganism producing the protein from the sample; the screening apparatus comprising a base plate having a plurality of micro-chambers arranged in the form of an array, and a fluorescence detector that detects the fluorescence produced on the base plate, wherein the fluorescence detector detects a protein having an enzymatic activity or a microorganism producing the protein that is present inside a micro-chamber by detecting the fluorescence produced by an enzyme reaction of the protein having an enzymatic activity with the fluorescent substrate.

In the apparatus for screening a protein or a microorganism, a protein having any enzymatic activity or a microorganism producing the protein is screened by using a base plate (micro-chamber array) having a plurality of micro-chambers arranged in the form of an array.

In other words, in the screening apparatus disclosed by the invention, a sample that can contain a protein having an enzymatic activity or a microorganism producing the protein and a fluorescent substrate producing fluorescence by hydrolysis are added onto a base plate having a plurality of micro-chambers arranged in the form of an array, and an enzyme reaction is induced between the protein having an enzymatic activity and the fluorescent substrate inside the micro-chamber. The fluorescence detector detects whether or not fluorescence is produced in a micro-chamber provided on the base plate, thereby determining the presence or absence of the enzyme reaction inside the micro-chamber and the presence or absence of the protein having an enzymatic activity or the microorganism producing the protein.

With such a screening apparatus, using a base plate having very small micro-chambers with a volume on the order of picoliters makes it possible to perform fast and inexpensive screening from a large-volume library. Furthermore, because each micro-chamber is extremely small, a solution comprising the target protein or a microorganism can be extracted from the sample in micro-amounts. As a result, in the case of microorganisms, one or several microorganisms can be taken out and they can also be caused to multiply. Therefore, microorganisms can be used in their existing state or in a multiplied state, for example, in the functional analysis performed at a stage following the screening. The library as referred to herein means all the proteins or all the microorganisms (including proteins and microorganisms other than the target proteins and microorganisms) that are contained in a sample.

The sample is thus a screening object. Specific examples of such samples include samples taken from the natural environment such as sea soil and samples containing mutant proteins or variants of microorganisms that are produced by a genetic transformation method based on conventional well-known genetic engineering technique.

When screening is conducted in accordance with the present invention using a sample taken from the natural environment such as sea soil, useful microorganisms having specific enzymatic activity can be screened from the natural world. Furthermore, using a sample containing mutant proteins or variants of microorganisms makes it possible to isolate easily the mutant proteins or variants produced from vast libraries.

In the screening apparatus described herein, the protein is a protein expressed in the surface layer of a cell membrane of a microorganism, and the fluorescent detector preferably detects the microorganism that expresses a protein having an enzymatic activity in the surface layer of a cell membrane by detecting a fluorescing micro-chamber from among a plurality of micro-chambers.

In other words, with the above-described screening method, the protein that is the object of screening is a protein expressed in the surface layer of a cell membrane of a microorganism such as yeast. Further, in this case the microorganism in which a protein having an enzymatic activity is expressed in the surface layer of a cell membrane is itself also included in the objects of screening.

If a sample containing a microorganism in which a protein is expressed in the surface layer of a cell membrane is dripped onto a base plate, the microorganism is distributed between a plurality of micro-chambers formed on the base plate. For this reason, the micro-chamber in which the target protein is contained can be verified by detecting a fluorescing micro-chamber from among a plurality of micro-chambers provided on the base plate, with the fluorescence detector.

The screening apparatus described herein preferably further comprises a micro-manipulator for taking out the contents of the micro-chamber where fluorescence is produced.

With such a configuration, a micro-amount of solution containing the target protein or microorganisms can be obtained. Despite an extremely small quantity thereof, the solution reliably contains the target protein or the microorganism and can be used as is, for e.g., in the functional analysis performed after the screening.

The inner diameter of the micro-chamber in the screening apparatus in accordance with the present invention is preferably 5 to 500 µm.

If the inner diameter of the micro-chamber is within the aforementioned range of 5 to 500 µm, an extremely small volume on the order of picoliters can be obtained for the micro-chamber. As a result, a solution containing a protein or a micro-organism contained in one micro-chamber can be used as is in the subsequently performed functional analysis.

Further, the method in accordance with the invention and apparatus described herein make it possible to check in an easy manner as to whether a protein having an enzymatic activity is expressed in the surface layer of a cell membrane or via secretion in a microorganism such as yeast or a cell. In order to examine whether or not a gene expressing the target protein in a microorganism or cell has been introduced, a technique is usually used by which an antibiotic-resistant gene is introduced into a vector and the possibility of growth in an antibiotic-added medium is verified. In this case an excess gene has to be bonded to a vector and this gene sometimes has to be removed.

On the other hand, when verification is performed using the method in accordance with the invention, a large amount of variants introduced from a small sample can be selected simultaneously. Furthermore, it is not necessary to introduce an excess gene into a vector. A method of introducing a gene of a light-emitting protein such as GFP and verifying the expression is performed as the usual method, but in this case, the excess gene also has to be bonded, whereby the vector length increases. The problem arising when the vector is long is that the gene is difficult to introduce or that the introduced gene does not express itself.

### EFFECTS OF THE INVENTION

As described herein above, the screening method in accordance with the invention and screening apparatus described herein use a base plate having extremely small micro-chambers with a volume on the order of picoliters, whereby fast and inexpensive screening can be performed from samples comprising a large-volume library. Furthermore, because each micro-chamber is extremely small, a micro-amount of solution containing the target protein or the microorganism can be extracted from the sample. As a result, the solution can be used as is, for example, in the functional analysis performed at a stage following the screening.

Thus, because the screening method in accordance with the invention and screening method described herein enable the implementation of a high-speed low-cost screening and also make it possible to obtain the target protein or the microorganism in a state facilitating the use thereof at the subsequent stage, these methods and apparatuses can be said to be highly convenient. In addition, the method in accordance with the invention and apparatus described herein make it possible to verify whether a microorganism expresses a protein having an enzymatic activity in the surface layer of a cell membrane or expresses a protein having an enzymatic activity by secretion.

### BRIEF DESCRIPTION OF THE DRAWINGS

[Fig. 1] Fig. 1 is a schematic drawing illustrating an example of the configuration of the screening apparatus described herein.
[Fig. 2] Fig. 2(a) is a schematic drawing illustrating an example of a base plate having micro chambers; Fig. 2(b) and Fig. 2(c) are schematic drawings illustrating a pattern of micro-chambers arranged on the base plate shown in Fig. 2(a).
[Fig. 3] Fig. 3(a) is a schematic drawing illustrating the results obtained in Example 1; Fig. 3 (b) is a schematic drawing illustrating the results obtained in Example 2; Fig. 3(c) is a schematic drawing illustrating the results obtained in Example 3.

### EXPLANATION OF REFERENCE NUMERALS

- 1: Base plate having a plurality of micro-chambers
- 2: Electric stage
- 3: CCD camera
- 4: PC
- 10: Screening apparatus

### BEST MODE FOR CARRYING OUT THE INVENTION

The invention is explained below in greater detail.

The screening of proteins or microorganisms in accordance with the invention uses a slide glass (base plate) in which micro-chambers with an inner diameter of 20 to 500 µm are arranged in the form of an array. The screening uses a technique according to which a sample comprising a yeast or a microorganism and a fluorescent substrate is introduced into micro-chambers, whereby an enzyme reaction is induced, and the fluorescence produced is observed with a fluorescent microscope or picked up with a CCD camera attached to a microscope, thereby detecting a micro-chamber where the enzyme reaction is initiated.

The screening method in accordance with the invention and the screening apparatus disclosed herein will be described below.

### (1) Screening method in accordance with the invention

The screening method in accordance with the invention comprises the steps of adding a fluorescent substrate onto a base plate having a plurality of micro-chambers arranged in the form of an array, dripping a sample comprising a protein or a microorganism onto the base plate, and detecting a protein having an enzymatic activity or a microorganism producing such a protein that is present inside the micro-chambers by detecting fluorescence emitted by an enzyme reaction of the protein or a microorganism and the fluorescent substrate.

The screening method uses a base plate having a plurality of micro-chambers arranged in the form of an array. This plate is called a micro-chamber array and has chambers in the form of extremely small recesses with a volume of about 1 to 2000 pL (for example, when the inner diameter of a micro-chamber is 500 µm, the volume is 2 nL, and when the inner diameter is 100 µm, the volume is 80 pL) that are arranged in the form of an array on a slide glass. Because this base plate is identical to a base plate having a plurality of micro-chambers that is provided in the screening apparatus described herein, it will be explained in greater detail in the description of the screening apparatus.

Then, a fluorescent substrate is added and a sample comprising a protein or a microorganism is dripped into the micro-chambers of the base plate. A step of adding the fluorescent substrate and a step of dripping the sample containing a protein or a microorganism is performed in no particular order and any one of them may be performed before the other. The "sample containing a protein or a microorganism" means the sample that is the object of screening. In the screening method in accordance with the present invention, it means that a protein having an enzymatic activity or a microorganism producing the protein is screened.

"The step of detecting a protein having an enzymatic activity or a microorganism producing such a protein that is present inside the micro-chamber" is performed after "the step of adding a fluorescent substrate" and "the step of dripping the sample comprising a protein or a microorganism". In the step of detecting, a micro-chamber in which an enzyme reaction has occurred is detected from among a plurality of micro-chambers arranged in the form of an array. The micro-chamber where the enzyme reaction has occurred is assumed to contain the target protein or a microorganism, and the micro-chamber where the enzyme reaction has not occurred is considered to contain no target protein or the microorganism.

The screening method in accordance with the invention uses a fluorescent substrate as an enzyme reaction substrate for verifying the presence of an enzyme reaction. Therefore, in the aforementioned step of detecting, whether or not the enzyme reaction has occurred can be easily verified by detecting whether the fluorescence was produced for each micro-chamber. For this reason, it can be easily determined as to whether the target protein or the microorganism is contained in the micro-chamber.

With the screening method in accordance with the invention, using a substrate having extremely small micro-chambers with a volume on the order of picoliters makes it possible to perform fast and inexpensive screening from samples comprising a large-volume library. Furthermore, because each micro-chamber is extremely small, a micro-amount of solution comprising the target protein or the microorganism can be extracted from the sample. As a result, in the case of microorganisms, one or several microorganisms can be taken out and they can also be caused to multiply. Therefore, microorganisms can be used in their existing state or in a multiplied state, for example, in the functional analysis performed at a stage following the screening.

Further, with the screening method in accordance with the invention, a take-out step of taking out the contents of a micro-chamber where the target protein is contained may be further added after the step of detecting. The take-out step can be implemented by using a micro-manipulator. By including such a take-out step, a micro-amount of solution containing the target protein or the microorganisms can be obtained. Despite an extremely small quantity thereof, the solution reliably contains the target protein or the microorganism and can be used as is, for e.g., in the functional analysis performed at a stage following the screening.

### (2) Screening apparatus as described herein.

The screening apparatus described herein is an apparatus for screening a protein having an enzymatic activity and a microorganism producing the protein from the sample. This screening apparatus comprises a base plate having a plurality of micro-chambers arranged in the form of an array and a fluorescence detector that detects the fluorescence produced on the base plate, wherein the fluorescence detector detects a protein having an enzymatic activity or a micro-organism producing the protein that is present inside a micro-chamber by detecting fluorescence produced by an enzyme reaction of the protein having an enzymatic activity with the fluorescent substrate.

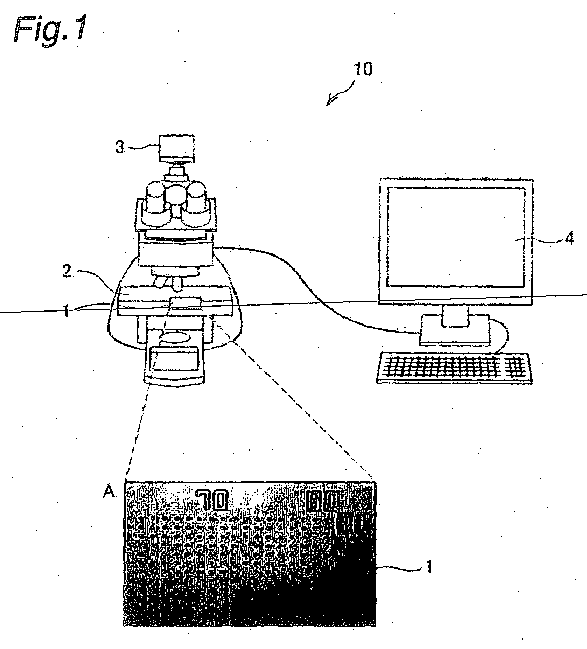

Fig. 1 shows an example of the configuration of the screening apparatus described herein. A screening apparatus 10 shown in Fig. 1 comprises a base plate 1 having micro-chambers, an electric stage 2 for carrying and moving the base plate 1, and a fluorescent detector for detecting fluorescence provided on the base plate 1. In the base plate 1, micro-chambers with an inner diameter of about 5 to 500 µm are arranged in the form of an array. Fig. 1A shows a chamber array disposed on the base plate 1. The fluorescence detector comprises a CCD camera 3 and a PC4 for displaying an image picked up by the CCD camera. The components constituting the screening apparatus will be described below

First, the base plate having a plurality of micro-chambers that is provided in the screening apparatus will be described.

Glass or a polycarbonate can be used as the base plate material. The micro-chambers may be formed by directly processing the base plate, or a film, for e.g., of polydimethylsiloxane or polyimide with wells (orifices) provided therein may be pasted on the base plate. When such a film is pasted, the wells opened in the film become micro-chambers. Alternatively, a film is coated on a base plate made from glass and micro-chambers are then formed by processing the film surface.

In such a screening apparatus, in order to detect the enzyme reaction of the target protein and substrate, it is preferred that the base plate and the film produce no fluorescence. Such micro-chamber arrays are presently available on the market.

For the micro-chambers formed on the substrate to have an appropriate volume inside the chambers, the inner diameter of the micro-chamber is preferably 5 to 500 µm, more preferably 10 to 100 µm. The depth of the micro-chamber is preferably 5 to 50 µm, more preferably 7 to 20 µm. Thus, if the inner diameter of a micro-chamber is within a range of 5 to 500 µm, the volume thereof can be about 1 to 2000 pL. Such a micro-amount of a solution is used in the functional analysis of proteins performed at a stage following the screening. Therefore, if the micro-chamber volume is set within the above-described range, a solution comprising a protein or a micro-organism that is contained in one micro-chamber can be used as is in the functional analysis performed at a stage following the screening.

The micro-chambers are arranged on a base plate with a width and length in a range of 5 to 20 mm, preferably 5 to 10 mm. The number of micro-chambers formed on one base plate is preferably 1000 to 100,000, more preferably 5000 to 100,000.

No specific limitation is placed on the shape of a micro-chamber, provided that it is concave. For example, the chamber may be in the form of a quadrangular pyramid, rectangular column, round column, hemisphere, and cone, as described in Non-patent document 1. Further, when the micro-chamber is in the form of a quadrangular pyramid or a rectangular column, "the inner diameter" mentioned hereinabove means a length of one side of the micro-chamber on the base plate surface. Furthermore, when the micro-chamber is in the form of a round column, a hemisphere, or a cone, "the inner diameter" mentioned hereinabove means the diameter of the micro-chamber at the base plate surface.

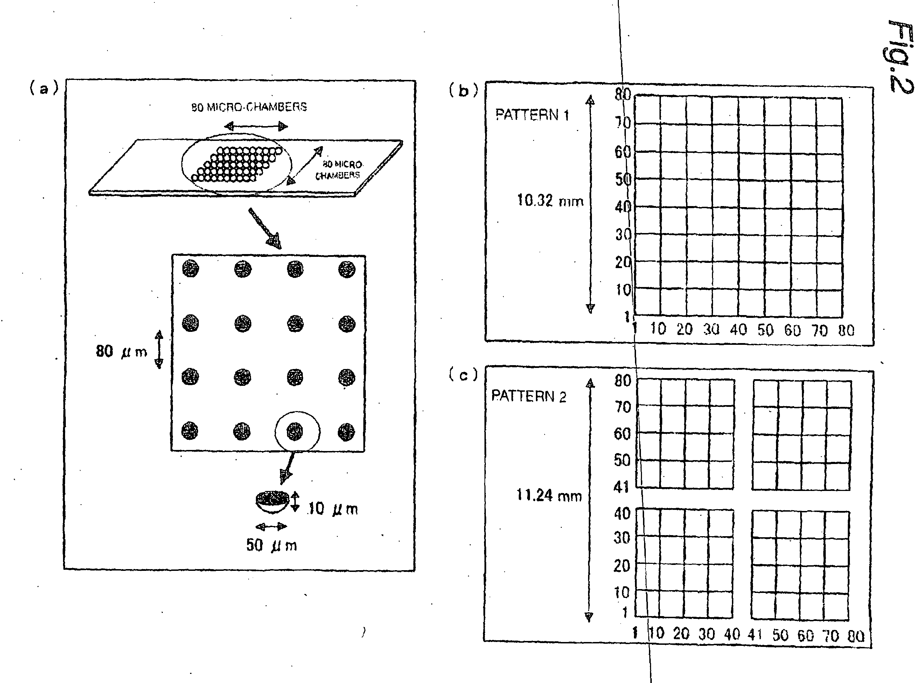

Fig. 2(a) is a schematic drawing illustrating an example of the substrate having a plurality of micro-chambers. The drawing in the upper section of Fig. 2(a) shows the entire base plate, and the drawing in the lower section is an enlarged view of the micro-chamber disposed on the base plate.
A total of 80 × 80 micro-chambers are disposed in almost the central section of the base plate shown in the figure in the form of an array with a spacing of 80 µm. The micro-chamber has a hemispherical shape, an inner diameter of 50 µm and a depth of 10 µm.

Fig. 2(b) and Fig. 2 (c) show an example of an array pattern of micro-chambers arranged on the base plate. In a pattern 1 shown in Fig. 2(b), 80 × 80 micro-chambers are arranged equidistantly within a range of 10.32 mm along one side. In a pattern 2 shown in Fig. 2(c), sectional configurations of 40 × 40 micro-chambers are arranged in twos along one side for a total of four configurations within a range of 11.24 mm along one side.

A photo-detector provided in the screening apparatus will be described below.

Any conventional well-known device capable of detecting the fluorescence produced from a detection object can be used as the fluorescence detector. Specific examples of such devices include fluorescence microscopes, and image display devices equipped with a light source and a CCD camera. The fluorescence detector in the screening apparatus shown in Fig. 1 is composed of a CCD camera 3 and a PC 4.

The screening apparatus described herein may comprise only the above-described base plate having micro-chambers and a fluorescence detector, but may be additionally equipped with a micro-manipulator for taking out the contents of the fluorescing micro-chamber.

If a micro-manipulator is provided, a micro-amount of solution containing the target protein or the microorganism can be obtained; and this micro-amount of solution can be used as is, for e.g., for the subsequently performed functional analysis of proteins.

A procedure of screening the target protein or the microorganism by using the screening apparatus described herein will be described below.

When screening is performed by using the above-described screening apparatus, first, a sample that can contain a protein or a microorganism that is the object of screening is dripped onto the base plate having micro-chambers formed therein.

When the sample is dripped, it scatters so that 1 to 100, preferably 1 to 10 microorganisms are introduced into one micro-chamber. Accordingly, it is preferred that a sample be prepared by suspending a library comprising yeast variants or microorganisms in water or a buffer to a turbidity of 0.01 to 1.0. A turbidity of about 0.5 is suitable for introducing one or several microorganisms into the chamber, but the turbidity level increases when a larger number of microorganisms is to be introduced into the micro-chambers. The number of microorganisms that enter a chamber correlates with the concentration of microorganisms contained in the sample and is assumed to follow a Poisson distribution.

Then, a solution containing a fluorescent substrate is added onto the base plate.

For example, when lipase activity is detected, a fatty acid ester of umbelliferone or a fatty acid ester of fluorescein can be used as the fluorescent substrate. Such substrates produce fluorescence when the esters are hydrolyzed. When glucosidase is detected, a glucoside derivative of umbelliferone, a glucoside derivative of fluorescein, or a glucoside derivative of resorfin can be used. Further, galactosidase and glucuronidase can be detected by using the same glucoside derivative or glucuronide derivative.

Protease comprising peptidase can be detected by using a substrate in which a fluorescent probe having an amino group such as an aminochroman derivative is amino bonded to a C terminal of an oligopeptide having an amino acid sequence of the substrates. For example, asparagyl chroman can be used at the C-terminal for caspases, lysyl chroman or arginyl chroman can be used at the C-terminal for proteases that recognize basic amino acids such as trypsin, and oligopeptides having phenyl alanyl chroman, leucyl chroman, and the like at the C-terminal can be used for proteases that recognize lipophilic amino acids such as chymotrypsin.

These fluorescent substrates have a property of producing fluorescence when they are hydrolyzed by catalytic action of enzymes. By detecting this fluorescence, the fluorescence detector can determine whether or not a protein having an enzymatic activity (for example, lipase activity, glucosidase activity, and protease activity) or a microorganism producing such a protein is present in the sample and can also determine the micro-chamber that contains the protein or the microorganism from among the micro-chambers arranged in the form of an array.

The substrate concentration in the solution containing the fluorescent substrate is preferably 10 µM to 1 mM. With the concentration at such a level, the enzymatic activity of the protein contained in the sample can be sufficiently detected.

As described herein above, if a sample and a solution containing a fluorescent substrate are added onto a base plate, an enzyme reaction is induced between the protein having an enzymatic activity that is contained in the sample and the fluorescent substrate, and the fluorescent substrate is hydrolyzed. By observing the fluorescence of a substance produced by the hydrolysis of the fluorescent substrate with the fluorescence microscope (fluorescence detector) provided for detecting the fluorescence produced on the base plate, the micro-chamber having the enzymatic activity can be detected. As a result, it is possible to detect the micro-chamber in which the target protein or the micro-organism is contained. Further, when the fluorescence detector is a PC equipped with a light source and a CCD camera, the base plate is irradiated with a light of an excitation wavelength from the light source, the fluorescence produced from the base plate is introduced into the PC with the CCD camera, and the base substrate producing the fluorescence is shown at an image display device provided in the PC. By observing the base plate on the image display device, the user can recognize the fluorescing micro-chamber.

When screening is performed by using the above-described apparatus, the step of dripping the sample and the step of adding the fluorescent substrate are performed in no particular order. In other words, instead of first dripping the sample, as described hereinabove, the fluorescent substrate solution can be added first and then the sample can be dripped and the enzymatic activity can be detected.

When the screening apparatus is provided with a micro-manipulator, a micro-amount of the target microorganism or protein having an enzymatic activity can be obtained by taking out the contents from the micro-chamber where the fluorescence is produced. As a result, in the case of microorganisms, one or several microorganisms can be taken out and they can also be caused to multiply. Therefore, the target microorganisms can be used in their existing state, for example, for the functional analysis.

### EXAMPLES

The invention will be described below in greater detail based on examples thereof.

[Example 1] Detection of hydrolysis reaction using a fluorescent substrate in a micro-chamber array in yeast that expresses lipase by secretion
In this example, yeast that expresses lipase by secretion was prepared by the following procedures.

### (1) Preparation of yeast that expresses lipase by secretion

A polymerase chain reaction (PCR) was performed by using EcoRI at 5' of a Geotrichum candidum lipase lip1 gene and using a primer with a SalI site at 3'. The PCR product obtained was cut at EcoRI, SalI, and then a plasmid pYEGCL1 that expresses Geotrichium candidum lipase by secretion was constructed by bonding with a secretive vector PYE22m for yeast that was similarly treated with EcoRI, SalI. The base sequence of the Geotrichum candidum lipase gene portion of the constructed pYEGCL1 was confirmed by the deoxyterminator method to have no errors. The yeast that expresses lipase by secretion was produced by genetic transformation of Saccharomyces cerevisiae EH1315 strain by pYEGCL1. To verify the plasmid introduction, the gene extracted from the genetically transformed strain was taken as a template and the PCR was performed by using primers at both ends of the Geotrichum candidum lipase gene. The verification was made by the amplification of a band close to a Geotrichum candidum lipase gene size of 1.6 kb.

### (2) Detection of hydrolysis reaction using a fluorescent substrate in a micro-chamber array

The yeast that expresses Geotrichum candidum lipase by secretion and was prepared by the above-described procedure was subjected to shaking incubation for 2 days at 30°C in 10 mL of a synthetic medium. The cell was then centrifugally separated for 10 min at 3000 rpm and 4°C and harvested. The cell thus obtained was suspended in 10 mL of PBS, then centrifugally separated under the same conditions, and harvested. The cell was washed one more time and suspended in 1 mL of PBS. The turbidity was measured at OD₆₀₀. A total of 40 µL of suspended cell (equivalent to OD₆₀₀ = 0.6) was then scattered over a micro-chamber array (inner diameter 20 µm) maintained at 4°C, and the array was allowed to stay for 10 min at 4°C. A total of 360 µL of a substrate (0.1 mM 4-methylumbelliferyl oleate/l% dimethylformamide/50 mM phosphoric acid buffer (pH 7.0)) was then added so as to be unified with the liquid surface and the system was allowed to stay for 10 min at 4°C. A cover glass was placed in a sliding manner, water was squeezed out, the plate was then set in an incubator (room temperature) at a 100% humidity, and a reaction was conducted for 1 h. The observations were then performed with a fluorescent microscope (excitation wavelength 320 nm, detection wavelength 450 nm).

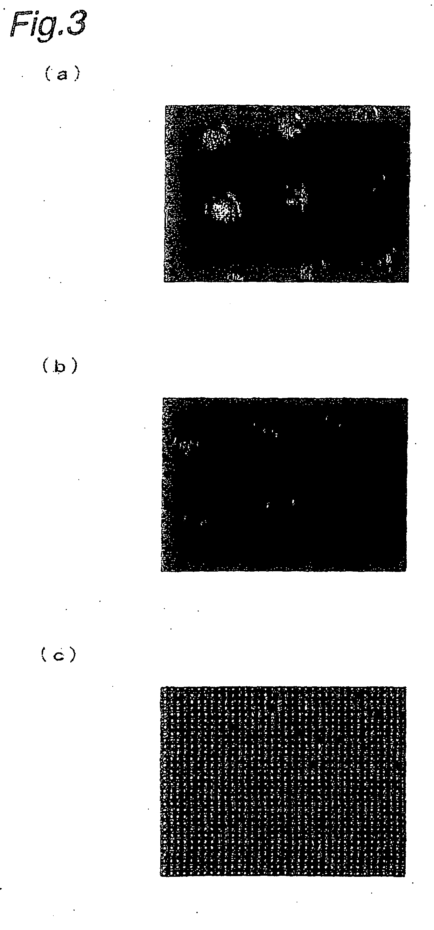

The results are shown in Fig. 3(a). This figure shows a microphotograph obtained with a magnification of 400. As shown in the figure, fluorescence was observed in each micro-chamber on the base plate and the yeast that expresses Geotrichum candidum lipase by secretion was confirmed to be present in each micro-chamber.

### [Example 2] Detection of hydrolysis reaction using a fluorescent substrate in a micro-chamber array in yeast that expresses lipase in the surface layer

In this example, first, yeast that expresses lipase in the surface layer was prepared by the following procedure

### (1) Preparation of yeast that expresses lipase (Geotrichum candidum lipase) in the surface layer

A polymerase chain reaction (PCR) was performed by using EcoRI at 5' of a Geotrichum candidum lipase lip1 gene and using a primer with a XhoI site at 3'. The PCR product obtained was cut at EcoRI, XhoI, and then a plasmid pCASS25GCL1 that expresses Geotrichium candidum lipase in the surface layer was constructed by bonding with a secretive vector pCASS25 for yeast that was similarly treated with EcoRI, SalI. The base sequence of the Geotrichum candidum lipase gene portion of the constructed pCASS25GCL1 was confirmed by the deoxyterminator method to have no errors. The yeast that expresses lipase in the surface layer was produced by genetic transformation of Saccharomyces cerevisiae EH1315 strain by pCASS25GCL1. To verify the plasmid introduction, the gene extracted from the genetically transformed strain was taken as a template and the PCR was performed by using primers at both ends of the Geotrichum candidum lipase gene. The verification was made by the amplification of a band close to a Geotrichum candidum lipase gene size of 1.6 kb.

### (2) Detection of hydrolysis reaction using a fluorescent substrate in a micro-chamber array

The yeast that expresses Geotrichum candidum lipase in the surface layer and was prepared by the above-described procedure was subjected to shaking incubation for 2 days at 30°C in 10 mL of a synthetic medium. The cell was then centrifugally separated for 10 min at 3000 rpm and 4°C and harvested. The cell thus obtained was suspended in 10 mL of PBS, then centrifugally separated under the same conditions, and harvested. The cell was washed one more time and suspended in 1 mL of PBS. The turbidity was measured at OD₆₀₀. A total of 40 µL of suspended cell (equivalent to OD₆₀₀ = 0.6) was then scattered over a micro-chamber array (inner diameter 20 µm) maintained at 4°C, and the array was allowed to stay for 10 min at 4°C. A total of 360 µL of a substrate (0.1 mM 4-methylumbelliferyl oleate /1% dimethylformamide/50 mM phosphoric acid buffer (pH 7.0)) was then added so as to be unified with the liquid surface and the system was allowed to stay for 10 min at 4°C. A cover glass was placed in a sliding manner, water was squeezed out, the plate was then set in an incubator (room temperature) at 100% humidity, and a reaction was conducted for 1 h. The observations were then performed with a fluorescent microscope (excitation wavelength 320 nm, detection wavelength 450 nm).

The results are shown in Fig. 3(b). This figure shows a micro-photograph obtained with a magnification of 400. As shown in the figure, fluorescence was observed in each micro-chamber on the base plate and the yeast that expresses Geotrichum candidum lipase in the surface layer was confirmed to be present in each micro-chamber.

### [Example 3] Detection of hydrolysis reaction using a fluorescent substrate in a micro-chamber array in marine microorganisms

A marine microorganism of Terrabacter sp. sampled from the sea sediment (reference: a bacterium with a 100% homology with 16SrRNA of Terrabacter sp. described in J. Bacteriol 179, 53-62, 1997) was subjected to shaking incubation for 5 days at 25°C in 10 mL of a commercial marine broth (Difco, cat no. 279110). The cell was then centrifugally separated for 5 min at 8000 rpm and 4°C and harvested. The cell thus obtained was suspended in 10 mL of PBS, then centrifugally separated under the same conditions, and harvested. The cell was washed one more time and suspended in 1 mL of PBS. The turbidity was measured at OD₆₀₀. A total of 40 µL of suspended cell (equivalent to OD₆₀₀ = 0.6) was then scattered over a micro-chamber array (inner diameter 100 µm) maintained at 4°C, and the array was allowed to stay for 10 min at 4°C. A total of 360 µL of a substrate (0.1 mM fluorescein dioleate/1% dimethylformamide/50 mM phosphoric acid buffer (pH 7.0)) was then added so as to be unified with the liquid surface and the system was allowed to stay for 10 min at 4°C. A cover glass was placed in a sliding manner, water was squeezed out, the plate was then set in an incubator (room temperature) at a 100% humidity, and a reaction was conducted for 1 h. The observations were then performed (excitation wavelength 473 nm, detection wavelength 532 nm) with a commercial micro-array scanner (CRBIO^{™}IIe-FIPC).

The results are shown in Fig. 3(c). As shown in the figure, fluorescence was observed in each micro-chamber on the base plate and the marine microorganism producing a protein having a lipase activity was confirmed to be present in each micro-chamber.

### INDUSTRIAL APPLICABILITY

The invention enables rapid and inexpensive screening in microscopic amounts. Therefore, it can be effectively used for the functional analysis of a large number of genes or proteins in genomics or proteomics.

## Claims

1. A method for detecting a protein having enzymatic activity or a microorganism producing this protein, comprising the steps of: adding a fluorescent substrate that produces fluorescence by hydrolysis onto a base plate having a plurality of micro-chambers arranged in the form of an array; dripping a sample comprising a protein or a microorganism on the base plate; and detecting a protein having an enzymatic activity or a microorganism producing this protein that is present inside the micro-chambers by detecting fluorescence emitted due to an enzyme reaction of the protein or the microorganism and the fluorescent substrate, wherein each inner diameter of the micro-chamber is 5 to 500 µm, which corresponds to a volume of 1 to 2000 pL.

2. The method according to claim 1, wherein the protein is a protein expressed in a surface layer of a cell membrane of a microorganism; and in the step of detecting, the microorganism that expresses the protein having an enzymatic activity in a surface layer of a cell membrane is detected by detecting a fluorescing micro-chamber from among the plurality of micro-chambers.

3. The method according to any one of claims 1 to 2, further comprising a step of taking out contents of the micro-chamber where the protein is contained after the step of detecting.

## Patentansprüche

1. Verfahren zum Nachweis eines Proteins, welches enzymatische Aktivität hat, oder eines Mikroorganismus, der dieses Protein herstellt, umfassend die Schritte: Zugabe eines fluoreszierenden Substrates, das Fluoreszenz durch Hydrolyse auf einer Basisplatte produziert, die eine Vielzahl von Mikrokammern hat, welche in der Form eines Arrays angeordnet sind; Tropfen einer Probe umfassend ein Protein oder einen Mikroorganismus auf die Basisplatte; und Nachweis eines Proteins, welches eine enzymatische Aktivität hat, oder eines Mikroorganismus, der dieses Protein herstellt, welches im Inneren der Mikrokammern vorliegt, durch Nachweis der Fluoreszenz, die aufgrund einer enzymatischen Reaktion des Proteins oder des Mikroorganismus und des fluoreszierenden Substrats emittiert wird, wobei der innere Durchmesser der Mikrokammer jeweils 5 bis 500µm ist, welches einem Volumen von 1 bis 2000 pL entspricht.

2. Verfahren gemäß Anspruch 1, wobei das Protein ein Protein ist, welches in der Oberflächenschicht einer Zellmembran eines Mikroorganismus exprimiert wird; und in dem Nachweisschritt der Mikroorganismus, welcher das Protein, das eine enzymatische Aktivität hat, in der Oberflächenschicht der Zellmembran exprimiert, durch den Nachweis einer fluoreszierenden Mikrokammer unter der Vielzahl von Mikrokammern nachgewiesen wird.

3. Verfahren gemäß einem der Ansprüche 1 bis 2, ferner umfassend einen Schritt des Herausnehmens des Inhalts der Mikrokammer, wo das Protein nach dem Schritt des Nachweises enthalten ist.

## Revendications

1. Procédé de détection d'une protéine ayant une activité enzymatique ou d'un micro-organisme produisant cette protéine, comprenant les étapes consistant à : ajouter un substrat fluorescent qui produit une fluorescence par hydrolyse sur le plateau comportant une pluralité de microchambres agencées sous la forme d'un réseau ; faire tomber en goutte à goutte un échantillon comprenant une protéine ou un micro-organisme sur le plateau ; et détecter une protéine ayant une activité enzymatique ou un micro-organisme produisant cette protéine qui est présent ou présente à l'intérieur des microchambres en détectant la fluorescence émise due à une réaction enzymatique de la protéine ou du micro-organisme et du substrat fluorescent, où chaque diamètre interne de la micro chambre est de 5 à 500 µm, ce qui correspond à un volume de 1 à 2 000 pL.

2. Procédé selon la revendication 1, dans lequel la protéine est une protéine exprimée dans une couche de surface d'une membrane cellulaire d'un micro-organisme ; et dans l'étape de détection, le micro-organisme qui exprime la protéine ayant une activité enzymatique dans une couche de surface d'une membrane cellulaire est détecté en détectant une microchambre fluorescente parmi la pluralité de microchambres.

3. Procédé selon l'une quelconque des revendications 1 à 2, comprenant en outre une étape de retrait du contenu de la microchambre où la protéine est contenue après l'étape de détection.
